# EUROPEAN PATENT SPECIFICATION

(11) **EP 3 124 050 B1**
(45) Date of publication and mention of the grant of the patent: **06.03.2019**
(21) Application number: 16181923.0
(22) Date of filing: 29.07.2016
(51) Int. Cl.: A61K 47/36, A61K 9/51

(54) **DRUG COMPOSITION COMPRISING A CHITOSAN DERIVATIVE AND METHOD FOR PREPARING THE SAME**
ARZNEIMITTELZUBEREITUNG ENTHALTEND EIN CHITOSANDERIVATIV UND METHODE ZUR IHRER HERSTELLUNG
COMPOSITION MEDICAMENTEUSE COMPRENANT UN DÉRIVÉ DE CHITOSANE ET SON PROCÉDÉ DE PRÉPARATION

(30) Priority: 30.07.2015 TW 104124706
(43) Date of publication of application: 01.02.2017
(73) Proprietor: National Chiao Tung University, Hsinchu 300 (TW)
(72) Inventor: Huang, Wei-Ting, 300 Taiwan (TW); Lee, Yi-Chi, New Taipei City 22063 (TW); Liu, Dean-Mo, 300 Taiwan (TW)
(74) Representative: Manitz Finsterwald Patentanwälte PartmbB

(56) References cited:
- LIU T Y ET AL: "Novel pH-sensitive chitosan-based hydrogel for encapsulating poorly water-soluble drugs", ACTA BIOMATERIALIA, ELSEVIER, AMSTERDAM, NL, vol. 6, no. 4, 1 April 2010 (2010-04-01), pages 1423-1429, XP026921463, ISSN: 1742-7061, DOI: 10.1016/J.ACTBIO.2009.10.010 [retrieved on 2009-10-09]
- MIWA A ET AL: "Development of novel chitosan derivatives as micellar carriers of taxol", PHARMACEUTICAL RESEARCH, SPRINGER NEW YORK LLC, US, vol. 15, no. 12, 1 January 1998 (1998-01-01), pages 1844-1850, XP002974049, ISSN: 0724-8741, DOI: 10.1023/A:1011901921995
- LI Y ET AL: "The preparation and characterization of a novel amphiphilic oleoyl-carboxymethyl chitosan self-assembled nanoparticles", CARBOHYDRATE POLYMERS, APPLIED SCIENCE PUBLISHERS, LTD. BARKING, GB, vol. 83, no. 1, 1 January 2011 (2011-01-01), pages 130-136, XP027353815, ISSN: 0144-8617 [retrieved on 2010-09-28]
- TAN Y L ET AL: "Self-aggregated nanoparticles from linoleic acid modified carboxymethyl chitosan: Synthesis, characterization and application in vitro", COLLOIDS AND SURFACES. B, BIOINTERFACES, ELSEVIER, AMSTERDAM, NL, vol. 69, no. 2, 1 March 2009 (2009-03-01), pages 178-182, XP025962200, ISSN: 0927-7765, DOI: 10.1016/J.COLSURFB.2008.11.026 [retrieved on 2008-11-30]
- LARSSON MIKAEL ET AL: "Biomedical applications and colloidal properties of amphiphilically modified chitosan hybrids", PROGRESS IN POLYMER SCIENCE, vol. 38, no. 9, 12 July 2013 (2013-07-12), pages 1307-1328, XP028691051, ISSN: 0079-6700, DOI: 10.1016/J.PROGPOLYMSCI.2013.06.009

## Description

### FIELD OF THE INVENTION

The invention relates to drug compositions and preparation methods thereof and, particularly, to a drug composition encapsulating hydrophobic drug(s) and hydrophilic drug(s) with amphiphilic chitosan, and a method for preparing the same.

### BACKGROUND OF THE INVENTION

Chitin presents broadly in nature, which occurs in crustaceans (such as in the exoskeleton of shrimps, crabs and insects), microorganisms (cell wall of bacteria and mushrooms) and plants such as algae. Chitosan [poly(β-1,4-glucosamine)], also called chitose, is a non-uniform polymeric material formed by various degrees of deacetylation reaction of chitin. Chitosan is a copolymer having N-acetylglucosamine and N-glucosamine as structural units, and the content of the N-glucosamine structural unit in the copolymer is usually more than 60%. Chitosan becomes an important macromolecular biomedical material due to its advantages of good biocompatibility, non-toxicity, degradability in organisms (by lysozyme), low price, and abundant raw materials without the worries of supply shortage.

In conventional encapsulating techniques for drug carriers, more than two anticancer drugs (e.g., hydrophilic drugs and hydrophobic drugs) are encapsulated in multiple-shell layers to form nanoparticles. The nanoparticles become multiple-functional for cancer treatment, when they are linked to an antibody/protein/peptide/polysaccharide having specific recognition or with the addition of materials for image development. However, encapsulating two drugs in a manner of multiple-shell layers tends to generate problems, such as leakage of the drugs, poor encapsulating rate, complication of the process, etc. Furthermore, it is difficult to encapsulate hydrophilic and hydrophobic drugs at the same time, if a macromolecule self-assembly manner is utilized to encapsulate drugs.

Liu T.-Y. et al. "Novel pH-sensitive chitosan-based hydrogel for encapsulating poorly water-soluble drugs", Acta Biomaterialia G (2010), 1423-1429, relates to the influence of the degree of carboxymethyl and hexanoyl substitution on the pH-sensitive swelling behavior, drug release behavior, and antiadhesion behavior of Carboxymethyl-hexanoyl chitosan (CHC) hydrogels (cross-linked with genipin). Hydrophobic Ibuprofen (a poorly water-soluble therapeutic agent) has been used as a model drug.

Miwa A. et al.: "Development of novel chitosan derivatives as micellar carriers of taxol", Pharmaceutical Research, vol. 15, no. 12, 1998, 1844-1850, discloses a chitosan with lauryl groups attached to amino groups to provide the hydrophobic moieties and, carboxymethyl groups attached to hydroxy groups to provide the hydrophilic moieties (N-lauryl-carboxymethyl-chitosan = LCC). The solubility of taxol - which is a hydrophobic drug - in LCC micelles in aqueous solution was examined.

Li Y. et al.: "The preparation and characterization of a novel amphiphilic oleoyl-carboxymethyl chitosan self-assembled nanoparticles", Carbohydrate Polymers 83 (2011), 130-136, describes that oleoyl-carboxymethyl chitosan (OCMCS) was synthesized by reacting carboxymethyl chitosan (CMCS) with oleoyl chloride and that nanoparticles were prepared using an o/w emulsification method. Formation of self-aggregation was observed by use of hydrophobic pyrene as a fluorescent probe in the OCMCS aqueous solution. Preparation of OCMCS nanoparticles was conducted by (i) dissolving OCMCS in distilled water, (ii) methylene chloride was added to the solution with stirring and homogenized, (iii) the solution was held under vacuum to remove methylene chloride and then (iv) sodium tripoly-phosphate (STPP) solution was added as a crosslinking reagent, (iv) the hydrophobic rifampicin loaded nanoparticles were prepared by dissolving rifampicin in methylene chloride and subsequently emulsifying as described above.

Tan Y. et al.: "Self-aggregated nanoparticles from linoleic acid modified carboxymethyl chitosan: Synthesis, characterization and application in vitro", Colloids and Surfaces B.: Biointerfaces, vol. 69 (2009), 178-182, describes the formation of linoleic acid (LA) modified carboxymethyl chitosan (LCC) and preparation of respective nanoparticles loaded with one single hydrophobic drug like adriamycin (ARD).

M. Larsson et al.: "Biomedical applications and colloidal properties of a amphiphilically modified chitosan hybrids" Progress in Polymer Science 38 (2013), 1307-1328, provides an overview regarding chitosan which has been chemically modified to selfassemble into nanoarchitectures that are usable in advanced biomedical applications, such as drug nanocarriers, macroscopic injectables. tissue-engineering scaffolds, and nanoimaging agents. It refers to colloidal amphiphilically modified chitosan (AMC) which may be loaded with a single drug or compound and merely states that a chitosan can be loaded with a hydrophilic moiety or a hydrophobic moiety.

Therefore, it is an important issue to resolve the process problems of requiring multiple steps and several environments in conventional nanoparticle encapsulation of multiple drugs by providing a method for encapsulating hydrophilic and hydrophobic drugs synchronously in a single step to prepare drug-carrying particles.

### SUMMARY OF THE INVENTION

The present invention provides a method for preparing a drug composition, which comprises: dispersing an amphiphilic chitosan, at least a hydrophobic drug, and at least a hydrophilic drug in a solvent to form a mixture
solution, wherein the amphiphilic chitosan was modified with a plurality of hydrophilic groups and a plurality of hydrophobic groups, and the pH of the mixture solution is adjusted to a range without precipitating the hydrophilic drug and the hydrophobic drug; and after stirring the mixture solution for at least 12 hours, receiving the drug composition when pH of the mixture solution is between 6 to 7.

The present invention further provides a drug composition which includes: an amphiphilic chitosan of formula (II) 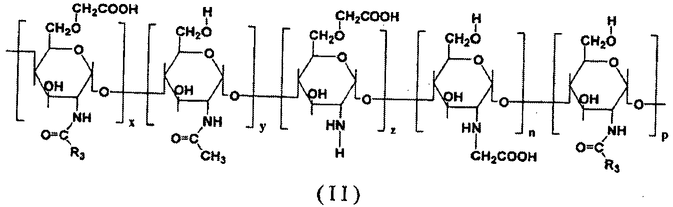 wherein, each R3 is independently a C5-C11 alkyl, and each of x, y, z, n and p is independently an integer between 20 to 2,000 modified with a plurality of hydrophilic groups and a plurality of hydrophobic groups; at least a hydrophilic drug embedded in the amphiphilic chitosan, and the at least a hydrophilic drug attracts the a plurality of hydrophilic groups by electrostatic force; and at least a hydrophobic drug embedded in the amphiphilic chitosan and gathered among the a plurality of hydrophobic groups.

From the foregoing, in the present invention, a proposal of encapsulating one or more same/different hydrophilic/hydrophobic drugs synchronously is achieved by using an amphiphilic chitosan macromolecular having high biocompatibility as a base and adjusting the solution environment for the macromolecular self-assembly of the amphiphilic chitosan. In addition, the surface of the drug-encapsulating particles is modified by attaching a targeting subject having specific identification capability (such as antibodies) through a cross-linking agent, or alternatively, a compound having image development effect can be encapsulated simultaneously with the drugs, thereby the nanoparticles become medical means with functions of identification, imaging and treatment to poison and kill cancer cells efficiently.

### BRIEF DESCRIPTION OF THE DRAWINGS

Fig. 1 shows encapsulating rates for Cisplatin (CDDP) and demethoxycurcumin (DMC) of double-drug-encapsulating nanoparticles in solutions at various pH values (pH 7.5, pH 8.5, pH 9.5, pH 10.5 and pH 11);
Fig. 2 shows encapsulating rates for Cisplatin and demethoxycurcumin of double-drug-encapsulating nanoparticles in solutions at various pH values (pH 8, pH 8.5, and pH 9);
Fig. 3 shows a transmission electron microscopic image of nanoparticles encapsulating double drugs;
Fig. 4 shows influences of three different drug/carrier combinations, i.e., free DMC-CDDP combination, CHC/DMC-CDDP nanoparticles and CHC/DMC-CDDP/Anti-CD133 nanoparticles, on cell survival rate; and
Fig. 5 shows influences of CHC/DMC-CDDP nanoparticles and CHC/DMC-CDDP/Anti-CD133 nanoparticles on cell survival rate, wherein X-axis shows contents of DMC and CDDP in the nanoparticles.

### DETAILED DESCRIPTION

The implementation of the present invention will be illustrated by the particular embodiments below; a person skilled in the art can readily understand the advantages and effects of the present invention by the content disclosed by the present specification. The present invention also can be performed or applied by other different modes, and details of the present specification also can be modified or altered based on various views or applications without departing from the spirit described in the present invention.

As used in the specification, the term "one-pot synthesis" means a method, in which reactant (e.g., amphiphilic chitosan) is allowed to encapsulate hydrophilic and hydrophobic drugs in one self-assembly reaction to improve reaction efficiency. The method can eliminate the lengthy processes for isolation and post-purification treatment, thereby shortening time, saving resources and improving efficiency.

The present invention provides a preparation method of a drug composition, which includes: dispersing an amphiphilic chitosan derivative, at least one hydrophobic drugs, and at least one hydrophilic drugs in a solvent such as water to form a mixture solution, wherein the amphiphilic chitosan derivative was modified with a plurality of hydrophilic groups and a plurality of hydrophobic groups, and the pH of the mixture solution is adjusted to a range without precipitating the hydrophilic drug and the hydrophobic drug; and after stirring the mixture solution for 12 hours, receiving the drug composition when pH of the mixture solution is between 6 to 7.

According to an embodiment of the present invention, the mixture solution was stirred for 12 to 24 hours.

According to an embodiment of the present invention, in the preparation method of the present invention, weight percentages range of the amphiphilic chitosan derivative is 0.01 wt% to 2 wt%, the concentration of the hydrophobic drug used is in a range of more than 0 to 3 mM, and the concentration of the hydrophilic drug used is in a range of more than 0 to 3 mM.

The hydroxyl group (-OH) and the amino group (-NH₂) on chitosan are sites which can be modified very easily. In general, chitosan is used as a backbone, and its hydroxyl terminal is modified to be hydrophilic, and its amino terminal is modified to be hydrophobic. The modification methods will not be detailed since they are well known.

In an Example of the foregoing preparation method, pH of the mixture solution is adjusted to a range in which the hydrophilic drug and the hydrophobic drug will not precipitate. In general, the mixture solution is adjusted to be weakly basic, specifically, by adding aqueous alkaline solution, i.e., adding an aqueous alkaline solution with pH above 8, for example, pH of 8.5 to 12.5, with pH of 9 to 10 being preferable, thereby, pH of the mixture solution is adjusted to a range in which the hydrophilic drug and the hydrophobic drug will not precipitate.

In an Example of the foregoing preparation method, first, a hydrophobic drug is added to an aqueous solution of amphiphilic chitosan derivative, then an aqueous alkaline solution is added thereto, at last, a hydrophilic drug is added, and pH of the mixture solution is adjusted to a range without precipitating the hydrophilic drug and the hydrophobic drug, wherein, the pH of the aqueous alkaline solution is in a range of 8.5 to 12.5 with pH of 9.0 to 10.5 being preferable.

Adjusting the mixture solution to be alkaline or adding a base can allow the H⁺ of a hydrophilic group on the biologic structure of the amphiphilic chitosan derivative to be released, and the hydrophilic group on the biologic structure of the amphiphilic chitosan derivative (such as COO⁻) will attract and conjugate a positively charged drug (such as Cisplatin, a hydrophilic drug) due to electrostatic force; and at the same time, since the hydrophilic drug is attracted by the electrostatic force, in contrast, a hydrophobic drug (such as demethoxycurcumin) will be encapsulated by hydrophobic groups (such as caproyl groups) on the biologic structure of the amphiphilic chitosan derivative; finally, a drug composition in the form of a plurality of particles is formed according to the self-assembly reaction.

After completing the encapsulation of drugs, the entire solution has a pH value of 6 to 7, with pH 6.7 being preferable, and the prepared drug-carrier particles are neutral and can be used without further neutralization reaction or purification step. According to an embodiment of the present invention, the drug composition is in the form of a plurality of particles. Additionally, the preparation method of the present invention further includes connecting a targeting subject to the surface of the plurality of particles using a cross-linking agent. In non-limiting Examples, the targeting subject is at least one selected from the group consisting of an antibody, a peptide and a protein.

In an embodiment, the cross-linking agent is at least one compound selected from the group consisting of 1-ethyl-(3-dimethylaminopropyl)carbodiimide (EDC) and hydroxysuccinimide.

According to an embodiment of the preparation method of the present invention, the method further includes dispersing an image developing compound in the solution, wherein the image developing compound is a fluorescent compound or an organometallic developer.

According to present invention, the chitosan derivative used in the present invention is showed in formula (II) below: 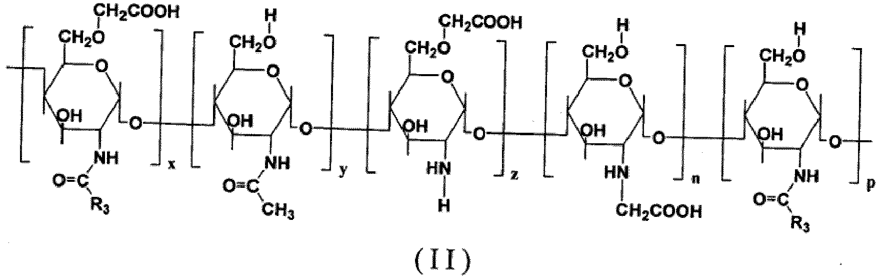 wherein, each R₃ is independently a C5-C11 alkyl, and each of x, y, z, n and p is independently an integer between 20 to 2,000.

According to an embodiment of the present invention, the hydrophobic drug used in the present invention is at least one selected from, but not limited to, the group consisting of Taxol, camptothecin, demethoxycurcumin (DMC), Topotecan, Cyclosporine A, Epirubicin and Rapamycin.

According to an embodiment of the present invention, the hydrophilic drug used in the present invention is at least one selected from, but not limited to, the group consisting of Cisplatin (CDDP), Doxorubicin, Oxaliplatin, Carboplatin, Nedaplatin and Satrapiatin.

The present invention further provides a drug composition which includes: an amphiphilic chitosan derivative modified with a plurality of hydrophilic groups and a plurality of hydrophobic groups; at least one hydrophilic drug embedded in the amphiphilic chitosan derivative, and the at least one hydrophilic drug attracts the a plurality of hydrophilic groups via electrostatic force; and at least one hydrophobic drug embedded in the amphiphilic chitosan derivative and gathered among the a plurality of hydrophobic groups.

According to an embodiment of the present invention, the drug composition is in the form of a plurality of particles. According to another embodiment of the present invention, the plurality of particles has a size in a range of 50 nm to 300 nm.

According to an embodiment of the present invention, the drug composition of the present invention is used for inhibiting the growth of cancer cells, wherein it is used for inhibiting the growth of at least one kind of cancer cells selected from the group consisting of non-small cell lung cancer cells, ovarian cancer cells, testicular cancer cells, bladder cancer cells, cervical cancer cells and lung cancer cells.

According to an embodiment of the present invention, the solvent and water can be removed from the drug composition of the present invention by means of lyophilization, concentration under reduced pressure, and vacuum drying.

The present invention will be further detailed with the Examples below, and it should be understood that the Examples is for illustration only.

### EAXMPLES

### Example 1: Preparation of amphiphilic chitosan derivatives modified with hydrophobic caproyl and hydrophilic carboxymethyl

First, 5 g of chitosan (Mw=215,000 g/mol, deacetyl degree of 80 to 90%, purchased from Adrich-Sigma) was suspended in isopropanol (50 mL) at room temperature, and the mixture was stirred for 30 minutes. The obtained suspension was mixed slowly with aqueous sodium hydroxide solution (12.5 mL) to form a mixture solution, and the grafting amount of hydrophilic functional groups was controlled by adjusting the concentration of sodium hydroxide in the mixture solution. Herein, the mixture solution contains 13.3 M of sodium hydroxide. Next, the mixture solution was allowed to react with chloroacetic acid to prepare a water-soluble hydrophilic carboxymethyl-modified chitosan which was then dried.

2 g of dried hydrophilic carboxymethyl-modified chitosan was dissolved in pure water (50 mL) and the solution was stirred for 24 hours. Next, the obtained solution was mixed with methanol (50 mL), and 0.2 M of caproic anhydride was added thereto. After reacting at room temperature for 20 hours, the reaction solution was collected and was dialyzed with aqueous ethanol solution (25% v/v) for 24 hours, then the product was collected after drying to obtain both hydrophobic caproyl-modified and hydrophilic carboxymethyl-modified amphiphilic chitosan derivative with a structure as shown by formula (II) below. Meanwhile, ¹H NMR spectrum and elemental analysis for N content were performed to determine the sites of substituents and caproyl grafting amount in the chitosan derivative. In this Example, grafting amount of caproyl is 13%.

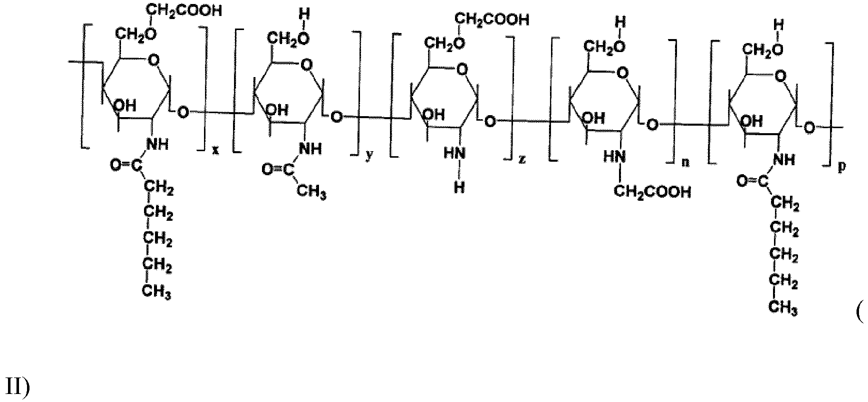

### Example 2: Encapsulating drugs using an amphiphilic chitosan derivative

0.5 mg of amphiphilic chitosan powder prepared in Example 1 was weighed and added to 200 µL of 0.1% demethoxycurcumin (in methanol), the mixture was mixed for a while using a shaker, 540 µL of double-distilled water was added thereto following by 60 µL of double-distilled water with pH 10.5, and 200 µL of 0.1% Cisplatin (in water) was added finally, and the mixture was stirred for 12 hours to prepare nanoparticles encapsulating double drugs (containing demethoxycurcumin and Cisplatin).

### Example 3: Test for encapsulating rate of drugs in nanoparticles encapsulating double drugs

The synthesized nanoparticles encapsulating double drugs were placed in a concentrating centrifuge tube, and perform centrifugation at a speed of 4,000 rpm for 30 minutes.

### (1). Encapsulating rate of Cisplatin

The clear solution in the concentrating centrifuge tube was diluted 20 folds and mixed with 0.14% solution of o-phenylenediamine in dimethylformamide at a ratio of 1:1, the mixture was heated at 100°C for 30 minutes and then was cooled in a refrigerator at -20°C for 10 minutes, finally, the solution was examined for the peak value at a wavelength of 705 nm with UV-visual spectrum, and content of non-encapsulated Cisplatin was converted from the peak value, thereby giving the encapsulating rate of Cisplatin.

### (2). Encapsulating rate test of demethoxycurcumin

The nanoparticles on the filter membrane of the concentrating centrifuge tube were re-dissolved in double-distilled water, the solution was mixed with methanol at a ratio of 1:1, and the mixture was analyzed using HPLC. The determination was performed at a flow rate of 1 mL/min under the detection condition at a wavelength of 425 nm, using C18 as stationary phase and acetonitrile-0.3% FA (50:50, v/v) as mobile phase, and the detection result was converted into the concentration of demethoxycurcumin to obtain the encapsulating rate of the drug.

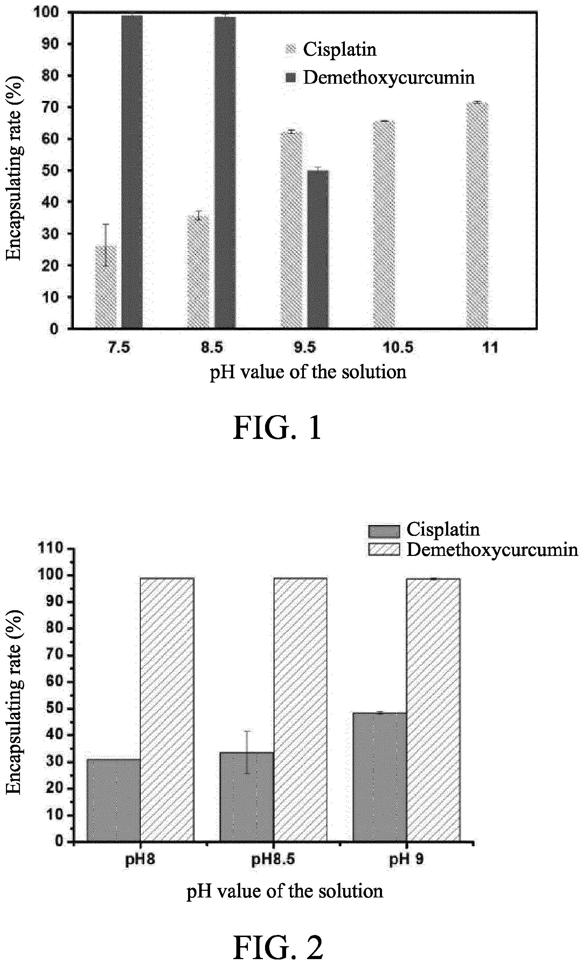

As shown in Fig. 1, the result showed that the encapsulating rate of Cisplatin increased as pH of the aqueous solution which is a mixture of the amphiphilic chitosan derivative, the hydrophilic drug and the hydrophobic drug increased, while the encapsulating rate of demethoxycurcumin decreased as pH of the solution increased. Since demethoxycurcumin trends to lose its medical activity and therefore precipitates in alkaline environment, it is preferable to adjust pH value of the aqueous solution to a pH between 8.0 to 9.0.

As shown in Fig. 2, the result showed that the best encapsulating rate of double drugs was gained when the aqueous solution had a pH value of 9.0, and pH of the entire solution after encapsulation was determined to be 6.7.

### Example 4: Conjugating an antibody to the nanoparticles encapsulating double drugs

A proper amount of antibody (anti-CD 133) and 0.01% 50 µL of cross-linking agent were added into the solution of nanoparticles encapsulating double drugs, the mixture was stirred for 5 hours to connect the antibody to the surface of the nanoparticles encapsulating double drugs described above, and the prepared nanoparticles encapsulating double drugs have diameter of 190 nm. The diameters and zeta potential of amphiphilic chitosan nanoparticles (hereinafter referred as CHC), nanoparticles encapsulating double drugs (hereinafter referred as CHC/DMC-CDDP), and antibody-conjugated nanoparticles encapsulating double drugs (hereinafter referred as CHC/DMC-CDDP-antibody) are shown in Table 1.

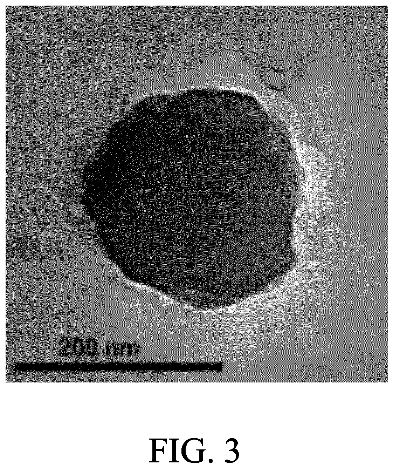

As shown in Fig. 3, the antibody-conjugated nanoparticles encapsulating double drugs were further observed through emission electron microscope.

**Table 1. Diameters and zeta potentials of various nanoparticles of the present invention**

| **Sample name** | **Diameter (nm)** | **Zeta potential (mV)** |
|---|---|---|
| CHC | 55±2.3 | -22.5±0.91 |
| CHC/DMC-CDDP | 140±7.3 | -13.0±3.5 |
| CHC/DMC-CDDP-Antibody | 190±6.1 | -5.1±0.6 |

### Example 5: Test for cells survival rate in poisoning and killing cancer cells with CHC/DMC-CDDP and CHC/DMC-CDDP-antibody nanoparticles of the present invention

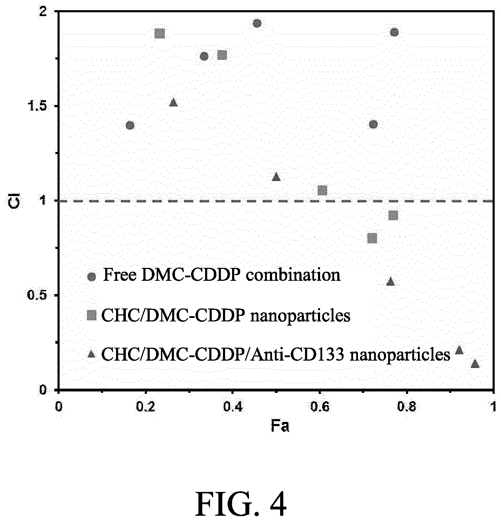

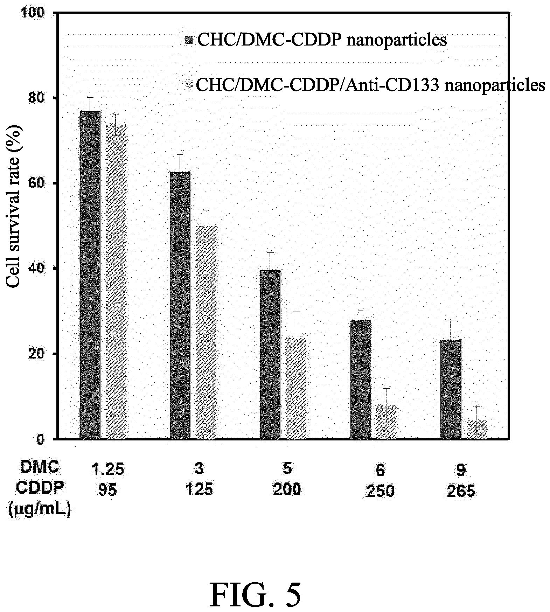

Lung cancer stem cells (A549-ON) were cultured in 24-well plate at 1×10⁵ cells per well, various amounts of formulated free DMC-CDDP combination (free drug) (from left to right in Fig. 4, the amounts were 3.25-75 g/mL, 7.5-150 g/mL, 7.5-300 g/mL, 15-300 g/mL and 20-600 g/mL), CHC/DMC-CDDP nanoparticles (from left to right in Fig. 4, the amounts were 1.25-95 g/mL, 3-125 g/mL, 5-200 g/mL, 6-250 g/mL and 9-265 g/mL) and CHC/DMC-CDDP/anti-CD133 nanoparticles (from left to right in Fig. 4, the amounts were 1.25-95 g/mL, 3-125 g/mL, 5-200 g/mL, 6-250 g/mL and 9-265 g/mL) were applied to the lung cancer stem cells, after culturing for 24 hours, the cells were analyzed using 3-(4,5-Dimethylthiazol-2-yl)-2,5-diphenyltetrazolium bromide (hereinafter referred as MTT) for cells survival rate. Fig. 4 shows effects of 3 kinds of different drug/carrier combination on the cell survival rate. As shown in Fig. 4, Y-axis is combination index (hereinafter referred as CI), the CI value of less than 1 represents the occurrence of synergistic effect; Fa represents cell apoptosis rate, and the closer to 1 the Fa value is, the higher cell apoptosis rate occurs, which represents better poisoning and killing effects on cancer cells. It can be seen in Fig. 4, drugs without being encapsulated in CHC (i.e., free DMC-CDDP combination) has no synergistic effect, since poisoning and killing effects on cancer cells cannot be enhanced by using double drugs at the same time; the double drugs encapsulated with CHC have clearly synergistic effects at certain amounts (CHC/DMC-CDDP: 6-250 g/mL and 9-265g/mL; CHC/DMC-CDDP/anti-CD133:5-200 g/mL, 6-250 g/mL and 9-265 g/mL) and provide good poisoning and killing effects on cancer cells. Fig. 5 shows effects of CHC/DMC-CDDP nanoparticles and CHC/DMC-CDDP/anti-CD133 nanoparticles on cell survival rate, i.e., effects of nanoparticles formed by CHC encapsulating double drugs with/without conjugating an antibody on poisoning and killing lung cancer stem cells. As shown in Fig. 5, using CHC/DMC-CDDP/anti-CD133 nanoparticles (antibody-conjugated) can effectively reduce survival rate of lung cancer stem cells.

## Claims

1. A preparation method of a drug composition, comprising:
dispersing an amphiphilic chitosan of formula (II) 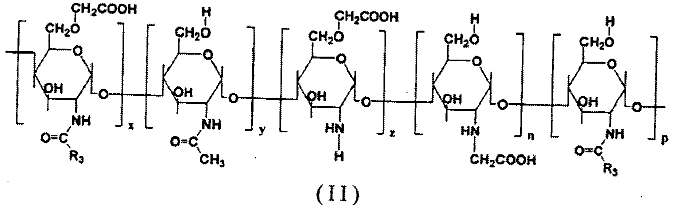 wherein, each R₃ is independently a C5-C11 alkyl, and each of x, y, z, n and p is independently an integer between 20 to 2,000, at least a hydrophobic drug, and at least a hydrophilic drug in an solvent to form a mixture solution, and the pH of the mixture solution is adjusted to a range without precipitating the hydrophilic drug and the hydrophobic drug; and
after stirring the mixture solution for at least 12 hours, receiving the drug composition when the pH of the mixture solution is between 6 to 7.

2. The preparation method of Claim 1, wherein, by adding an aqueous solution with pH of 8.5 to 12.5, the pH of the mixture solution is adjusted to a range without precipitating the hydrophilic drug and the hydrophobic drug.

3. The preparation method of Claim 2, wherein, by adding an aqueous solution with pH of 9.0 to 10.5, the pH of the mixture solution is adjusted to a range without precipitating the hydrophilic drug and the hydrophobic drug.

4. The preparation method of Claim 1, wherein the drug composition is in the form of a plurality of particles.

5. The preparation method of Claim 4, further comprising coupling a targeting subject to surfaces of the plurality of particles via using a cross-linking agent.

6. The preparation method of Claim 5, wherein the targeting subject is at least one selected from the group consisting of an antibody, a peptide and a protein.

7. The preparation method of Claim 1, further comprising dispersing an image developing compound in the solution.

8. The preparation method of Claim 7, wherein the image developing compound is a fluorescent compound or an organometallic developer.

9. A drug composition comprising:
an amphiphilic chitosan of formula (II) 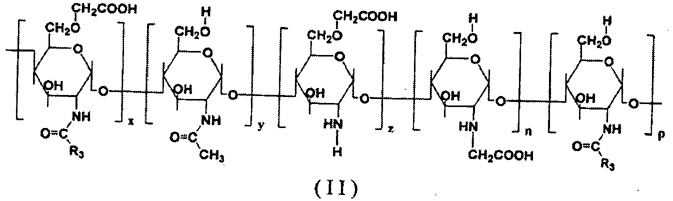 wherein, each R₃ is independently a C5-C11 alkyl, and each of x, y, z, n and p is independently an integer between 20 to 2,000
at least a hydrophilic drug embedded in the amphiphilic chitosan, and the at least a hydrophilic drug attracting the plurality of hydrophilic groups by electrostatic force; and
at least a hydrophobic drug embedded in the amphiphilic chitosan and gathered among the plurality of hydrophobic groups.

10. The drug composition of Claim 9, wherein the drug composition is in the form of a plurality of particles.

11. The drug composition of Claim 10, further comprising a cross-linking agent for a targeting subject coupled to surfaces of the plurality of particles via the cross-linking agent.

12. The drug composition of Claim 11, wherein the targeting subject is at least one selected from the group consisting of an antibody, a peptide and a protein.

13. The drug composition of Claim 10, wherein the particle size of the plurality of particles is in a range of from 50 nm to 300 nm.

14. The drug composition of Claim 9, which is used for inhibiting growth of cancer cells.

15. The drug composition of Claim 14, which is used for inhibiting the growth of at least one kind of cancer cells selected from the group consisting of non-small cell lung cancer cells, ovarian cancer cells, testicular cancer cells, bladder cancer cells, cervical cancer cells and lung cancer cells.

## Patentansprüche

1. Herstellungsverfahren für eine Arzneimittelzubereitung, umfassend:
Dispergieren eines amphiphilen Chitosans der Formel (II), 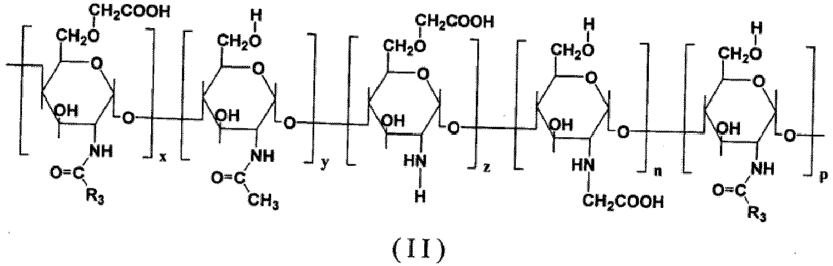 wobei jedes R₃ unabhängig voneinander ein C5-C11-Alkyl ist, und jedes x, y, z, n und p unabhängig voneinander eine ganze Zahl zwischen 20 und 2000 ist, mindestens eines hydrophoben Arzneimittels und mindestens eines hydrophilen Arzneimittels in einem Lösungsmittel, um eine gemischte Lösung zu bilden,
und wobei der pH-Wert der gemischten Lösung auf einen Bereich eingestellt wird, ohne das hydrophile Arzneimittel und das hydrophobe Arzneimittel auszufällen;
und wobei, nach mindestens 12 Stunden Rühren der gemischten Lösung, die Arzneimittelzusammensetzung erhalten wird, wenn der pH-Wert der gemischten Lösung zwischen 6 und 7 liegt.

2. Herstellungsverfahren nach Anspruch 1, wobei durch Hinzufügen einer wässrigen Lösung mit einem pH-Wert von 8,5 bis 12,5 der pH-Wert der gemischten Lösung auf einen Bereich eingestellt wird, ohne das hydrophile Arzneimittel und das hydrophobe Arzneimittel auszufällen.

3. Herstellungsverfahren nach Anspruch 2, wobei durch Hinzufügen einer wässrigen Lösung mit einem pH-Wert von 9,0 bis 10,5 der pH-Wert der gemischten Lösung auf einen Bereich eingestellt wird, ohne das hydrophile Arzneimittel und das hydrophobe Arzneimittel auszufällen.

4. Herstellungsverfahren nach Anspruch 1, wobei die Arzneimittelzubereitung die Form einer Mehrzahl von Partikeln hat.

5. Herstellungsverfahren nach Anspruch 4, weiterhin umfassend das Verankern eines Zielgegenstands auf Oberflächen der Mehrzahl von Partikeln durch Verwendung eines Vernetzungsmittels.

6. Herstellungsverfahren nach Anspruch 5, wobei der Zielgegenstand mindestens eines ausgewählt aus der Gruppe bestehend aus einem Antikörper, einem Peptid und einem Protein ist.

7. Herstellungsverfahren nach Anspruch 1, weiterhin umfassend das Dispergieren einer Bildgebungsverbindung in der Lösung.

8. Herstellungsverfahren nach Anspruch 7, wobei die Bildgebungsverbindung eine fluoreszierende Verbindung oder ein metallorganischer Bildgeber ist.

9. Arzneimittelzubereitung umfassend:
ein amphiphiles Chitosan der Formel (II), 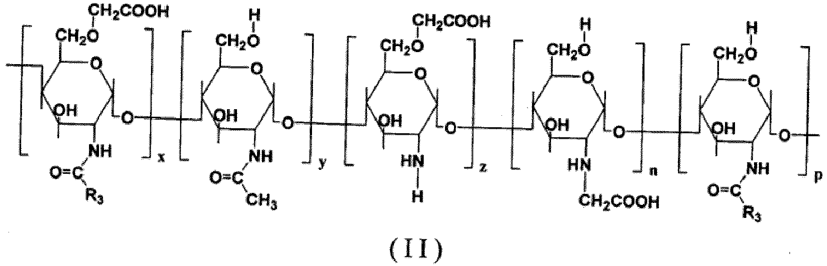 wobei jedes R₃ unabhängig voneinander ein C5-C11-Alkyl ist, und jedes x, y, z, n und p unabhängig voneinander eine ganze Zahl zwischen 20 und 2000 ist;
mindestens ein hydrophiles Arzneimittel, das in dem amphiphilen Chitosan eingebettet ist, wobei das mindestens eine hydrophile Arzneimittel die Mehrzahl hydrophiler Gruppen durch elektrostatische Kraft anzieht; und
mindestens ein hydrophobes Arzneimittel, das in dem amphiphilen Chitosan eingebettet ist und sich zwischen der Mehrzahl hydrophober Gruppen sammelt.

10. Arzneimittelzubereitung nach Anspruch 9, wobei die Arzneimittelzubereitung in Form einer Mehrzahl von Partikeln vorliegt.

11. Arzneimittelzubereitung nach Anspruch 10, weiterhin umfassend ein Vernetzungsmittel für einen Zielgegenstand, der durch das Vernetzungsmittel auf Oberflächen der Mehrzahl von Partikeln verankert ist.

12. Arzneimittelzubereitung nach Anspruch 11, wobei der Zielgegenstand mindestens eines ausgewählt aus der Gruppe bestehend aus einem Antikörper, einem Peptid und einem Protein ist.

13. Arzneimittelzubereitung nach Anspruch 10, wobei die Partikelgröße der Mehrzahl von Partikeln im Bereich von 50 nm bis 300 nm liegt.

14. Arzneimittelzusammensetzung nach Anspruch 9, die zur Hemmung des Wachstums von Krebszellen verwendet wird.

15. Arzneimittelzusammensetzung nach Anspruch 14, die verwendet wird zur Hemmung des Wachstums von mindestens einer Art von Krebszellen, ausgewählt aus der Gruppe bestehend aus nicht-kleinzelligen Lungenkrebszellen, Eierstockkrebszellen, Hodenkrebszellen, Blasenkrebszellen, Gebärmutterhalskrebszellen und Lungenkrebszellen.

## Revendications

1. Procédé de préparation d'une composition médicamenteuse, comprenant :
la dispersion d'un chitosane amphiphile selon la formule (II) 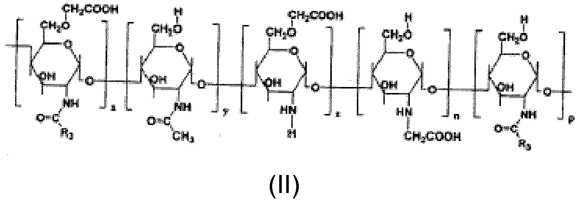 où, chaque R₃ représente indépendamment un groupement alkyle en C5 à C11, et chacun des x, y, z, n et p représente indépendamment un nombre entier entre 20 et 2 000, au moins un médicament hydrophobe, et au moins un médicament hydrophile dans un solvant pour former une solution de mélange, et le pH de la solution de mélange est ajusté dans une plage sans précipitation du médicament hydrophile et du médicament hydrophobe ; et
après agitation de la solution de mélange pendant au moins 12 heures, la collecte de la composition médicamenteuse lorsque le pH de la solution de mélange se situe entre 6 et 7.

2. Procédé de préparation selon la revendication 1, dans lequel, par ajout d'une solution aqueuse avec un pH entre 8,5 et 12,5, le pH de la solution de mélange est ajusté dans une plage sans précipitation du médicament hydrophile et du médicament hydrophobe.

3. Procédé de préparation selon la revendication 2, dans lequel, par ajout d'une solution aqueuse avec un pH entre 9,0 et 10,5, le pH de la solution de mélange est ajusté dans une plage sans précipitation du médicament hydrophile et du médicament hydrophobe.

4. Procédé de préparation selon la revendication 1, dans lequel la composition médicamenteuse est sous la forme d'une pluralité de particules.

5. Procédé de préparation selon la revendication 4, comprenant en outre le couplage d'un objet de ciblage avec les surfaces de la pluralité des particules par l'intermédiaire de l'utilisation d'un agent de réticulation.

6. Procédé de préparation selon la revendication 5, dans lequel l'objet de ciblage est au moins un objet choisi parmi le groupe constitué par un anticorps, un peptide ou une protéine.

7. Procédé de préparation selon la revendication 1, comprenant en outre la dispersion d'un composé de développement d'une image dans la solution.

8. Procédé de préparation selon la revendication 7, dans lequel le composé de développement d'une image est un composé fluorescent ou un produit de développement organométallique.

9. Composition médicamenteuse, comprenant :
un chitosane amphiphile selon la formule (II) 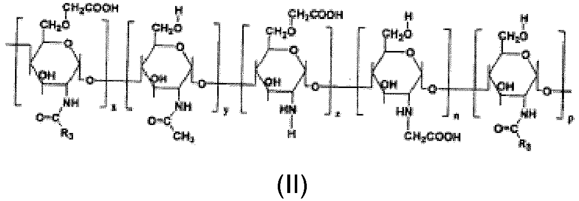 où, chaque R₃ représente indépendamment un groupement alkyle en C5 à C11, et chacun des x, y, z, n et p représente indépendamment un nombre entier entre 20 et 2 000
au moins un médicament hydrophile incorporé dans le chitosane amphiphile, et l'au moins un médicament hydrophile attirant la pluralité des groupements hydrophiles par la force électrostatique ; et
au moins un médicament hydrophobe incorporé dans le chitosane amphiphile et collecté parmi la pluralité des groupements hydrophobes.

10. Composition médicamenteuse selon la revendication 9, où la composition médicamenteuse est sous la forme d'une pluralité de particules.

11. Composition médicamenteuse selon la revendication 10, comprenant en outre un agent de réticulation destiné à un objet de ciblage couplé aux surfaces de la pluralité des particules par l'intermédiaire de l'agent de réticulation.

12. Composition médicamenteuse selon la revendication 11, où l'objet de ciblage est au moins un objet choisi parmi le groupe constitué par un anticorps, un peptide et une protéine.

13. Composition médicamenteuse selon la revendication 10, où la taille particulaire de la pluralité des particules se situe dans une plage allant de 50 nm à 300 nm.

14. Composition médicamenteuse selon la revendication 9, qui est utilisée pour inhiber la croissance de cellules cancéreuses.

15. Composition médicamenteuse selon la revendication 14, qui est utilisée pour inhiber la croissance d'au moins un type de cellules cancéreuses choisies parmi le groupe constitué par les cellules cancéreuses du poumon non à petites cellules, les cellules cancéreuses de l'ovaire, les cellules cancéreuses du testicule, les cellules cancéreuses de la vessie, les cellules cancéreuses du col de l'utérus et les cellules cancéreuses du poumon.
